# EUROPEAN PATENT APPLICATION

(11) **EP 4 360 451 A1**
(43) Date of publication of application: **01.05.2024**
(21) Application number: 22306616.8
(22) Date of filing: 25.10.2022
(51) Int. Cl.: A01H 5/10, A01H 6/46

(54) **MUTANTS FOR HAPLOID INDUCTION**

(71) Applicant: Limagrain Europe, 63360 Saint Beauzire (FR); Institut national de recherche pour l'agriculture, l'alimentation et l'environnement, 75007 Paris (FR); Centre National de la Recherche Scientifique, 75016 Paris (FR); Ecole Normale Supérieure de Lyon, 69007 Lyon (FR); Université Claude Bernard Lyon 1, 69100 Villeurbanne (FR)
(72) Inventor: WIDIEZ, Thomas, 69002 LYON (FR); ROGOWSKY, Peter, 69007 LYON (FR); GILLES, Laurine, 26290 DONZÈRE (FR); JACQUIER, Nathanael, 73190 CHALLES LES EAUX (FR); MARTINANT, Jean-Pierre, 63910 VERTAIZON (FR)
(74) Representative: Lavoix

(57) **Abstract**

The instant invention relates to haploid inducer plant lines and methods for producing these plant lines. The invention further relates to methods for producing a haploid plant, and to the use of such haploid plant for generating a doubled haploid plant.

## Description

The instant invention relates to haploid inducer plant lines and methods for producing these plant lines. The invention further relates to methods for producing a haploid plant, and to the use of such haploid plant for generating a doubled haploid plant.

Haploid inducer lines induce paternal and/or maternal haploids *in planta.* Maternal haploid induction is triggered by the pollinator (male) parent: crosses using pollen from such a haploid inducer line lead to the development of the egg cell into a haploid embryo containing solely the maternal chromosome set or genome, whereas the endosperm is normally fertilized. When inducers are used as the seed-bearing parent, they are called paternal haploid inducers, because only the nuclear genome of the paternal donor plant is passed to haploid embryos. However, in contrast to maternal haploid inducers, paternal inducers also transmit their cytoplasmic genome to haploid seeds, which may or may not be desirable (Trentin et al., 2020, Plants (Basel), 9(5): 614; Jacquier, N.M.A., et al.2020 Nat. Plants 6, 610-619).

Double haploid (DH) technology is a powerful way to improve plant breeding efficiency. DH lines are usually created *in vivo,* through crosses with maternal haploid inducers. Progenies of DH plants are genetically homogeneous material, allowing breeders to evaluate their traits of interest on genetically fixed material at an early stage of the breeding cycle, thus increasing breeding efficiency. The DH technology relies on two main steps: (1) a haploid induction system to generate haploid embryos or plantlets, and (2) a chromosome doubling step to restore diploidy of these plantlets that can be self-pollinated to produce completely homozygous seeds. This rapid method of inbred line production reduces the length of breeding cycles and, consequently, increases genetic gain.

DH breeding based on *in vivo* haploid induction has recently becomes even more attractive because it could be combined with gene editing to directly edit elite lines (Kelliher et al., Nat Biotechnol. 2019 Mar;37(3):287-292; Wang et al., Nat Biotechnol. 2019 Mar;37(3):283-286). Nevertheless, a major bottleneck is the availability of haploid induction systems to produce haploid plantlets. Intra-specific crosses represent an attractive way to induce haploid plants because the process is fully *in planta,* leading to seed-based haploid embryo formation without the need of *in vitro* embryos rescue (Jacquier et al. Nat. Plants, 2020,6, 610-619). Nevertheless, those so-called haploid inducers line are limited to very few plants and especially scarce in dicotyledon plants (Jacquier et al. Nat. Plants, 2020,6, 610-619). The identification of DOMAIN OF UNKNOWN FUNCTION 679 membrane proteins (DMP) as a molecular player of the maize haploid inducer line *in planta* haploid induction (Zhong et al., 2019, Nat Plants, 5(6):575-580) successfully allowed the translation of haploid induction property to the dicotyledon species: *Arabidopsis, Medicago truncatula* and tomato (Wang et al., 2022, Plant Biotechnology Journal, 20, 22-24.; Zhong et al., 2020, Nat. Plants, 6, 466-472; Zhong et al., 2022, Plant Biotechnology Journal, 20, 250-252). In addition of producing haploid embryos, knocking-out sperm cell orthologous *DMP* genes were found to lead to double fertilization defects and seeds abortions (Cyprys et al., 2019, Nature Plants, 5, 253-257; Takahashi et al., 2018, Development, 145, dev170076). More precisely, *AtDMP8,9* mutations lead to preferential single fertilization of the central cell underlying a role in gametes interaction.

The present invention aims at providing new haploid inducer plant lines by combining two mechanisms of haploid induction leading respectively to defective sperm cells and to promotion of fertilization of the central cell.

The inventors further identified that the *KPL* and *GEX2* genes are implicated in the process of fertilization of central cell and that mutation thereof lead to double fertilization defects, and haploid induction, similarly to *DMP* gene mutants.

### SUMMARY OF THE INVENTION

The invention relates to a haploid inducer plant line, wherein said haploid inducer plant line comprises at least
a) a first mutated gene causing defective sperm cell function;
b) a second mutated gene promoting fertilization of central cell; with the proviso that if the first mutated gene is the gene *MATRILINEAL*/*NOT LIKE*

*DAD*/*ZmPHOSPHOLIPASE-A1,* and the second mutated gene is *DMP* gene, then a third mutated gene is present causing defective sperm cell function or promoting fertilization of central cell.

The invention further relates to a haploid inducer plant line, wherein said plant line comprises a mutated *GEX2* and/or *KPL* gene that promotes fertilization of central cell.

The invention also relates to a method for producing a haploid inducer plant line according to the invention, wherein:
(a) mutated gene leads to the modulating of the expression of a gene in a plant, wherein the modulation of expression of said gene results in a loss of function of the gene and causes defective sperm cell function; or
(b) mutated gene leads to the modulating the expression of a gene in a plant, wherein the modulation of expression of said gene results in a loss of function of the gene and promotes fertilization of central cell;
(c) modulating the expression of *GEX2* and/or *KPL* gene in a plant, wherein the modulation of expression of *GEX2* and/or *KPL* gene results in a loss of function of the gene(s);
whereby said plant exhibit haploid induction activity.

The invention further relates to a method for producing a haploid plant, comprising:
(a) pollinating a female plant with pollen from a haploid inducer plant line according to the invention; and
(b) screening the progeny of pollinated female plant for selecting haploid plants.

Another method for producing a haploid plant according to the invention, comprises:
(a) exposing pollen from a haploid inducer plant line comprising a mutated gene promoting fertilization of central cell to an agent causing chromosome fragmentation;
(b) pollinating a female plant with said pollen exposed in (a) to the agent causing chromosome fragmentation;
(c) screening the progeny of pollinated female plant for selecting haploid plants.

Still another method for producing a haploid plant according to the invention, comprises:
(a) pollinating a female plant simultaneously, separately or successively with (i) pollen from a first haploid inducer plant line comprising a mutated gene causing sperm cell to contain defective chromosomes, and (ii) pollen from a second haploid inducer plant line comprising a mutated gene promoting fertilization of central cell;
(b) screening the progeny of the pollinated female plant for selecting haploid plants produced by heterofertilization.

In another aspect the invention relates to a method for generating a doubled haploid plant comprising inducing chromosome doubling in the haploid plant produced by a method of the invention.

### DETAILED DESCRIPTION

### Haploid inducer plant line

It is provided a haploid inducer plant line, wherein said haploid inducer plant line comprises at least:
a) a first mutated gene causing defective sperm cell function;
b) a second mutated gene promoting fertilization of central cell; with the proviso that if the first mutated gene is the gene *MATRILINEAL*/*NOT LIKE*

*DAD*/*ZmPHOSPHOLlPASE-A1,* and the second mutated gene is ZmDMPgene, then a third mutated gene is present causing sperm cell to contain defective chromosomes or causing defective double fertilization.

Sexually mature pollen (the male gametophyte) is composed of three haploid cells: a large vegetative cell that engulfs two sperm cells. The vegetative nucleus and the two sperm cells are connected in a stable association named the male germ unit (MGU). In the vegetative cell, the two sperm cells are encircled by a specific membrane derived from the plasma membrane of the pollen vegetative cell that is called pollen endo-plasma membrane (endo-PM). The MATRILINEAL/NOT LIKE DAD/ZmPHOSPHOLIPASE-A1 protein has been showed to localized to this endo-PM (Gilles L. et al. 2021, J Cell Biol, 220 (10): e202010077).

Double fertilization is the fertilization process in which the egg cell and central cell are fertilized by the two sperm cells of a pollen grain. The fertilized egg cell forms a diploid embryo and the fertilized central cell forms a triploid endosperm. When sperm cells derive from two different pollen grains, the process is called heterofertilization.

By "haploid" is meant a character related to cells or plants or parts of plants comprising such cells whose chromosomes contained in their nucleus are each in only one copy (n).

Defective male gametes can lead to failed egg cell-sperm cell fusion but normal central cell-sperm cell fusion leading to endosperm development and stimulation of the development of the haploid egg cell into a haploid embryo. Defective male gametes can also lead to the failure of double fertilization and the production of aneuploid embryos and/or endosperms, which abort.

By "haploid inducer plant" it is meant that the plant, e.g. when used as pollinator, leads to formation of haploid offspring at a significantly higher frequency than what is observed in natural populations of plant, the haploid embryo contains genetic material from the pollinated plant, these inducers are known as paternal haploid inducer and produce maternal haploid embryos. Some maternal haploid inducers are also well known, haploid obtained on these plants contains paternal genetic material. For a review see Jacquier, N.M.A., et al.2020 Nat. Plants 6, 610-619.

By "mutated gene causing defective sperm cell function" is it meant that the mutation in the gene results in that the sperm cells cannot achieve their normal function in plant double fertilization, i.e. achieve fertilization of (i) the egg cell to form a diploid embryo and/or (ii) the central cell to form a triploid endosperm. Preferably, the mutated gene causing defective sperm cell function impairs fertilization of the egg cell with a sperm cell but does not impair fertilization of the central cell with the other sperm cell. The defect caused by the gene mutation includes causing defective sperm cell, causing sperm cells to contain defective chromosomes (such as fragmented chromosomes, instable chromosomes), causing defective pollen with impairment of endo-plasma membrane (endo-PM) composition.

According to an embodiment, the mutation is a knock-out or loss-of-function mutation of the gene.

According to some embodiments, the first gene which mutation causes defective sperm cell function is selected from the group consisting of *NOT LIKE DAD* (*NLD*) gene, a gene encoding Phosphatidylinositol 4-phosphate 5-kinase, a gene encoding Phosphatidylinositol/phosphatidylcholine transfer protein, a gene encoding Phospholipase D, a gene which is Zm00001d042182 or an orthologous thereof, a gene encoding an Oleosin, a gene encoding Nucleic acid-binding OB-fold-like protein, a gene encoding beta-glucanase, a gene encoding Putative DEAD-box ATP-dependent RNA helicase family protein, a gene encoding Plasma membrane ATPase, a gene encoding NAD(P)H dehydrogenase, and a gene encoding p-loop nucleoside triphosphate hydrolase superfamily protein with Calponin Homology domain.

Preferably, the first gene which mutation causes defective sperm cell function is selected from the group consisting of *NLD* gene, a gene encoding Phosphatidylinositol 4-phosphate 5-kinase, and a gene encoding Phosphatidylinositol/phosphatidylcholine transfer protein.

Table 2 contains a non-exhaustive list of suitable first genes that can be mutated to cause defective sperm cell function, and thus haploid induction

**Table 1: Genes which mutation causes defective sperm cell function, and thus haploid induction.**

| **Maize gene ID V4** | **Maize gene ID V5** | **Putative Gene function/annotation** |
|---|---|---|
| Zm00001d047457 | Zm00001 eb394480 | PIP5K candidate |
| Zm00001d029211 | Zm00001 eb017290 | PIP5K paralogue |
| Zm00001d029209 | Zm00001 eb017280 | PIP5K paralogue |
| Zm00001d004955 | Zm00001 eb093110 | PIP5K paralogue |
| Zm00001 d042182 | Zm00001 eb141900 | Uncharacterized protein |
| Zm00001d015175 | Zm00001 eb231470 | Phosphatidylinositol/phosphatidylcholine transfer protein |
| Zm00001 d016601 | Zm00001 eb242010 | Phosphatidylinositol/phosphatidylcholine transfer protein |
| Zm00001 d013269 | Zm00001 eb214880 | Beta-glucanase |
| Zm00001d052022 | Zm00001eb192290 | Plasma membrane ATPase candidate |
| Zm00001d045122 | Zm00001 eb374840 | Plasma membrane ATPase paralogue |
| Zm00001 d014157 | Zm00001 eb222860 | NAD(P)H dehydrogenase / Flavoprotein wrbA |
| Zm00001 d043249 | Zm00001 eb151280 | Minor allergen / NAD(P)H dehydrogenase |
| Zm00001 d012607 | Zm00001 eb369750 | Minor allergen / NAD(P)H dehydrogenase |
| Zm00001d024533 | Zm00001eb415650 | Putative DEAD-box ATP-dependent RNA helicase family protein |
| Zm00001 d007712 | Zm00001 eb115940 | p-loop nucleoside triphosphate hydrolase superfamily protein with Calponin Homology domain |
| Zm00001 d048673 | Zm00001 eb165280 | p-loop nucleoside triphosphate hydrolase superfamily protein with Calponin Homology domain |
| Zm00001d037643 | Zm00001 eb283150 | Phospholipase D ZmPLD3 |
| Zm00001d005770 | Zm00001 eb099650 | Phospholipase D ZmPLD8 |
| Zm00001 d009648 | Zm00001 eb344420 | Oleosin Bn-V |
| Zm00001 d044080 | Zm00001 eb158660 | OLE-5 |
| Zm00001 d025132 | Zm00001 eb422630 | Nucleic acid-binding OB-fold-like protein 1 |
| Zm00001 d036151 | na | Nucleic acid-binding OB-fold-like protein 2 |

According to some embodiments, the second gene which mutation promotes fertilization of central cell is selected from the group consisting of *DMP, KPL,* and *GEX2* genes. According to an embodiment, the mutation is a knock-out or loss-of-function mutation of the gene. Table 2 contains a non-exhaustive list of suitable second genes.

**Table 2: genes which mutation promotes fertilization of central cell, and thus haploid induction**

| **Maize gene ID V4** | **Maize gene ID V5** | **Gene abbreviation** |
|---|---|---|
| Zm00001d044822 | Zm00001 eb372320 | ZmDMP |
| Zm00001d002427 | Zm00001 eb07181 0 | ZmKPLa |
| Zm00001 d026242 | Zm00001 eb430330 | ZmKPLb |
| Zm00001d005781 | Zm00001 eb099800 | ZmGEX |

In one embodiment, the first gene is *NOT LIKE DAD* (*NLD*) that encodes a predicted patatin-like phospholipase A (Gilles et al. 2017, EMBO J. 36, 707-717; Kelliher et al. 2017, Nature 542, 105-109; Liu et al., 2017, Mol. Plant 10, 520-522; international patent application published as WO2016/177887). A haploid inducer mutant of maize gene *NLD* comprises sequence SEQ ID NO:1 as described in WO2016/177887 (herein SEQ ID NO:27).

In one embodiment, the first gene encodes Phosphatidylinositol 4-phosphate 5-kinase, or PIP5K. In one embodiment, PIP5K denotes a gene encoding a polypeptide identified as ENZYME entry: EC 2.7.1.68. A sequence of maize *PIP5K* gene is available in maize genome assembly version Zm-B73-REFERENCE-GRAMENE-4.0 under entry Zm00001d047457, Zm00001d029211, Zm00001d029209, Zm00001d004955. A sequence of *Arabidopsis thaliana PIP5K* gene is available in The Arabidopsis Information Resource (TAIR) database under entries AT3G56960.1, At1g01460.1, At3g07960.1, and At2g41210.1 (all according to version of 2017-07-23). Preferably, the first gene encoding PIP5K comprises or consists of a polynucleotide encoding SEQ ID NO:1 (translation product of Zm00001d047457, as available in GenBank under accession: AQL06564), or an orthologue thereof which encodes a sequence at least 50% identical to SEQ ID NO:1.

In one embodiment, the first gene encodes Phosphatidylinositol/phosphatidylcholine transfer protein. A sequence of maize gene encoding Phosphatidylinositol/phosphatidylcholine transfer protein is available in maize genome assembly version Zm-B73-REFERENCE-GRAMENE-4.0 under entry Zm00001d015175 or Zm00001d016601. A sequence of *Arabidopsis thaliana* gene encoding Phosphatidylinositol/phosphatidylcholine transfer protein is available in AT2G21540, AT4G36490, AT4G39180, and AT2G18180. Preferably, the first gene encoding Phosphatidylinositol/phosphatidylcholine transfer protein comprises or consists of a polynucleotide encoding SEQ ID NO:2 (translation product of Zm00001d015175, as available in GenBank under accession: AQK68222), or an orthologue thereof which encodes a sequence at least 50% identical to SEQ ID NO:2. In particular, an haploid inducer mutant of maize gene encoding Phosphatidylinositol/phosphatidylcholine transfer protein comprises SEQ ID NO:21 (herein 'C5175_cri-2"), or SEQ ID NO:24 (herein 'P6601 - cri-1').

In one embodiment, the first gene encodes a Phospholipase D, such as maize PLD3 or PLD8. A sequence of maize *PLD3* is available in maize genome assembly version Zm-B73-REFERENCE-GRAMENE-4.0 under entry Zm00001d037643. A sequence of maize *PLD8* is available in maize genome assembly version Zm-B73-REFERENCE-GRAMENE-4.0 under entry Zm00001d005770. Preferably, the first gene encoding Phospholipase D comprises or consists of a polynucleotide encoding SEQ ID NO:3 (translation product of Zm00001d037643, as available in GenBank under accession: AQK84516), or an orthologue thereof which encodes a sequence at least 50% identical to SEQ ID NO:3. According to certain embodiments, the first gene does not encode a Phospholipase D, such as maize *PLD3* or *PDL8.*

In one embodiment, the first gene which is Zm00001d042182 or an orthologous thereof is a gene available in maize genome assembly version Zm-B73-REFERENCE-GRAMENE-4.0 under entry Zm00001d042182 or an orthologous thereof. Preferably, the first gene encodes a polypeptide comprising or consisting of SEQ ID NO:4 (translation product of Zm00001d042182, as available in GenBank under accession ONM34738), or an orthologue thereof which encodes a sequence at least 50% identical to SEQ ID NO:4.

In one embodiment, the first gene encodes an Oleosin, such as maize Oleosin Bn-V or Oleosin 5 (OLE-5). A sequence of maize *Oleosin Bn-V* is available in maize genome assembly version Zm-B73-REFERENCE-GRAMENE-4.0 under entry Zm00001 d009648. A sequence of maize *OLE-5* is available in maize genome assembly version Zm-B73-REFERENCE-GRAMENE-4.0 under entry Zm00001d044080. Preferably, the first gene encoding Oleosin Bn-V comprises or consists of a polynucleotide encoding SEQ ID NO:5 (translation product of Zm00001d009648, as available in GenBank under accession: AQK92342), or an orthologue thereof which encodes a sequence at least 50% identical to SEQ ID NO:5.

In one embodiment, the first gene encodes a nucleic acid-binding, OB-fold-like protein, such as maize nucleic acid-binding OB-fold-like protein 1 or nucleic acid-binding, OB-fold-like protein 2. A sequence of maize nucleic acid-binding OB-fold-like protein 1 is available in maize genome assembly version Zm-B73-REFERENCE-GRAMENE-4.0 under entry Zm00001d025132. A sequence of maize nucleic acid-binding OB-fold-like protein 2 is available in maize genome assembly version Zm-B73-REFERENCE-GRAMENE-4.0 under entry Zm00001 d036151. Preferably, the first gene encoding a nucleic acid-binding, OB-fold-like protein comprises or consists of a polynucleotide encoding SEQ ID NO:6 (translation product of Zm00001 d025132, as available in GenBank under accession: AQK43062), or an orthologue thereof which encodes a sequence at least 50% identical to SEQ ID NO:6.

In one embodiment, the first gene encodes beta-glucanase. A sequence of maize beta-glucanase is available in maize genome assembly version Zm-B73-REFERENCE-GRAMENE-4.0 under entry Zm00001d013269. Preferably, the first gene encoding beta-glucanase comprises or consists of a polynucleotide encoding SEQ ID NO:7 (translation product of Zm00001 d013269, as available in GenBank under accession: AQK62872), or an orthologue thereof which encodes a sequence at least 50% identical to SEQ ID NO:7.

In one embodiment, the first gene encodes Putative DEAD-box ATP-dependent RNA helicase family protein. A sequence of maize Putative DEAD-box ATP-dependent RNA helicase family protein is available in maize genome assembly version Zm-B73-REFERENCE-GRAMENE-4.0 under entry Zm00001d024533. Preferably, the first gene encoding Putative DEAD-box ATP-dependent RNA helicase family protein comprises or consists of a polynucleotide encoding SEQ ID NO:12 (translation product of Zm00001 d024533, as available in GenBank under accession: AQK41443), or an orthologue thereof which encodes a sequence at least 50% identical to SEQ ID NO:12.

In one embodiment, the first gene encodes Plasma membrane ATPase. A sequence of maize Plasma membrane ATPase is available in maize genome assembly version Zm-B73-REFERENCE-GRAMENE-4.0 under entry Zm00001d052022. Preferably, the first gene encoding Plasma membrane ATPase comprises or consists of a polynucleotide encoding SEQ ID NO:13 (translation product of Zm00001d052022, as available in GenBank under accession AQK55788), or an orthologue thereof which encodes a sequence at least 50% identical to SEQ ID NO:13.

In one embodiment, the first gene encodes NAD(P)H dehydrogenase. A sequence of maize NAD(P)H dehydrogenase is available in maize genome assembly version Zm-B73-REFERENCE-GRAMENE-4.0 under entry Zm00001d014157. Preferably, the first gene encoding NAD(P)H dehydrogenase comprises or consists of a polynucleotide encoding SEQ ID NO:14 (translation product of Zm00001d014157, as available in GenBank under accession AQK65363), or an orthologue thereof which encodes a sequence at least 50% identical to SEQ ID NO:14.

In one embodiment, the first gene encodes p-loop nucleoside triphosphate hydrolase superfamily protein with Calponin Homology domain. A sequence of maize p-loop nucleoside triphosphate hydrolase superfamily protein with Calponin Homology domain is available in maize genome assembly version Zm-B73-REFERENCE-GRAMENE-4.0 under entry Zm00001d007712. Preferably, the first gene encoding p-loop nucleoside triphosphate hydrolase superfamily protein with Calponin Homology domain comprises or consists of a polynucleotide encoding SEQ ID NO:15 (translation product of Zm00001d007712, as available in GenBank under accession ONM27393), or an orthologue thereof which encodes a sequence at least 50% identical to SEQ ID NO:15.

In one embodiment, the second gene is *DMP* gene, *such* as *DMP1* in maize or *DMP8 or DMP9* in *Arabidopsis thaliana.* A sequence of maize *DMP1* (DUF679 domain membrane protein 1) is available in maize genome assembly version Zm-B73-REFERENCE-GRAMENE-4.0 under entry Zm00001d044822. A sequence of *Arabidopsis thaliana DMP8 gene* is available in The Arabidopsis Information Resource (TAIR) database under entry AT1G09157. A sequence of *Arabidopsis thaliana DMP9* gene is available in The Arabidopsis Information Resource (TAIR) database under entry AT5G39650. Preferably, the *DMP* gene comprises or consists of a polynucleotide encoding ZmDMP1 (SEQ ID NO:8; (translation product of Zm00001d044822, as available in GenBank under accession: AQL00945), an orthologue thereof which encodes a sequence at least 50% identical to SEQ ID NO:8. According to certain embodiments, the second gene which mutation promotes fertilization of central cell denotes both *Arabidopsis thaliana DMP8* and *DMP9* genes as mutation of both genes is required for haploid induction.

In one embodiment, the second gene is *KPL* that encodes Protein KOKOPELLI. A sequence of maize KPLa is available in maize genome assembly version Zm-B73-REFERENCE-GRAMENE-4.0 under entry Zm00001d002427. A sequence of maize KPLb is available in maize genome assembly version Zm-B73-REFERENCE-GRAMENE-4.0 under entry Zm00001 d026242. A sequence of Arabidopsis thaliana Protein KOKOPELLI is available in The Arabidopsis Information Resource (TAIR) database under entry AT5G63720. Preferably, the second gene *KPL* comprises or consists of a polynucleotide encoding SEQ ID NO:9 (translation product of Zm00001d002427, as available in GenBank under accession: ONM14289), or an orthologue thereof which encodes a sequence at least 50% identical to SEQ ID NO:9. The second gene *KPL* mays also comprise or consist of a polynucleotide encoding SEQ ID NO:10 (translation product of AT5G63720), or an orthologue thereof which encodes a sequence at least 50% identical to SEQ ID NO:10. In particular, a mutant of maize KPLa comprises SEQ ID NO:16 (herein '*Zm*Pla cri-1"). Preferably, a mutant haploid inducer version of maize KPLb comprises SEQ ID NO:17, SEQ ID NO:18, SEQ ID NO:19 or SEQ ID NO:20 (herein *'Zmkp*/b cri-1' to 'Zmkplb cri-4').

In one embodiment, the second gene is *GEX2* that encodes protein GAMETE EXPRESSED 2. A sequence of maize *GEX2* is available in maize genome assembly version Zm-B73-REFERENCE-GRAMENE-4.0 under entry Zm00001d005781. A sequence of *Arabidopsis thaliana* protein *GEX2* is available in The Arabidopsis Information Resource (TAIR) database under entry AT5G49150. Preferably, the second gene comprises or consists of a polynucleotide encoding SEQ ID NO:11 (translation product of Zm00001d005781, as available in GenBank under accession: ONM22082), or an orthologue thereof which encodes a sequence at least 50% identical to SEQ ID NO:11.

By "orthologous sequence" is intended a gene or polypeptide having the same or a similar function in the same or different species. In particular, in the case of different species, it is meant homologous sequences that are inferred to be descended from the same ancestral sequence separated by a speciation event: when a species diverges into two separate species, the copies of a single gene in the two resulting species are said to be orthologous. In that case, orthologous genes are genes in different species that originated by vertical descent from a single gene of the last common ancestor.

When the sequence is contemplated in the same species, an orthologous sequence also meant homologous sequences that are inferred to be descended from the same ancestral sequence.

Orthologous sequences may be found by bioinformatics methods known by the man skilled in the art and the starting from the reference polynucleotide or sequence encoding the reference polypeptide invention, by searching for homologous sequences (e.g. sequences at least 50%, 55%, 60%, 65%, 70%, 75%, 80%, 85%, or 90% identical to the reference sequence) and by selecting as orthologous sequences encoding polypeptides sharing the same or a similar function with the reference polypeptide, or at least sharing structural features that are thought to be important for the function.

In the haploid inducer plant line, if the first mutated gene is the *NLD* gene (in particular maize *NLD* gene*)*, and the second mutated gene is DMPgene (in particular maize *DMP* gene), then a third mutated gene is present causing defective sperm cell function or promoting fertilization of central cell.

In that case, the third mutated gene may be selected from the group consisting of a gene encoding Phosphatidylinositol 4-phosphate 5-kinase, a gene encoding Phosphatidylinositol/phosphatidylcholine transfer protein, a gene encoding Phospholipase D, a gene which is Zm00001d042182 or an orthologous thereof, a gene encoding an Oleosin, a gene encoding a Nucleic acid-binding OB-fold-like protein, a gene encoding beta-glucanase, *KPL* gene, and *GEX2* gene. According to an embodiment, the third mutated gene is selected from the group consisting of Phosphatidylinositol 4-phosphate 5-kinase, and a gene encoding Phosphatidylinositol/phosphatidylcholine transfer protein, *KPL* gene, and *GEX2* gene*.*

According to some embodiments, the haploid inducer plant line does not comprise a mutated *NLD* gene (in particular mutated maize *NLD* gene*)*, nor a mutated *DMP* gene (in particular maize *DMP* gene).

Also provided is a haploid inducer plant line, wherein said plant line comprises a mutated *GEX2* and/or *KPL* gene that causes defective double fertilization, in particular that promotes fertilization of central cell.

According to an embodiment, said haploid inducer plant line comprises at least one mutation in a gene that encodes Protein KOKOPELLI, in particular in gene *KPLb.* Preferably, a mutant haploid inducer version of maize KPLb comprises SEQ ID NO:17, SEQ ID NO:18, SEQ ID NO:19 or SEQ ID NO:20 (herein *'Zmkp*/b cri-1' to 'Zmkplb cri-4'). Advantageoulsy, said haploid inducer plant line further comprises a mutation in the gene *NOT LIKE DAD* (*NLD*)*,* which preferably comprise of consist of SEQ ID NO:27. According to an embodiment, said haploid inducer plant line comprises a mutation in gene *KPLb* and a mutation in gene *NLD.* Preferably, said haploid inducer plant line is a maize haploid inducer plant line.

According to an embodiment, said haploid inducer plant line comprises at least a mutated gene *ZmKPLb* comprising or consisting of sequence SEQ ID NO:17, SEQ ID NO:18, SEQ ID NO:19, or SEQ ID NO:20, or an orthologue thereof (if the plant is other than maize). According to an embodiment, said haploid inducer plant line comprises at least a mutated gene *ZmKPLb* comprising or consisting of sequence SEQ ID NO:17, SEQ ID NO:18, SEQ ID NO:19, or SEQ ID NO:20, and a mutated gene NLD comprising or consisting of sequence SEQ ID NO:1, or an orthologue thereof (if the plant is other than maize).

According to an embodiment, said haploid inducer plant line comprises at least a mutated form of the gene encoding Phosphatidylinositol/phosphatidylcholine transfer protein A comprising or consisting of sequence SEQ ID NO:21, or an orthologue thereof (if the plant is other than maize). According to an embodiment, said haploid inducer plant line comprises at least a mutated form of the gene encoding Phosphatidylinositol/phosphatidylcholine transfer protein A comprising or consisting of sequence SEQ ID NO:21, and a mutated form of the gene encoding Phosphatidylinositol/phosphatidylcholine transfer protein B comprising or consisting of sequence SEQ ID NO:24, or an orthologue thereof (if the plant is other than maize).

**Table 3 : CRISPR/Cas9 generated mutants**

| **CRISPR plasmid** # | **Maize gene ID V4** | **Internal Name** |
|---|---|---|
| L2126 | Zm00001d002427 | ZmKPLa |
| L2126 | Zm00001d026242 | ZmKPLb |
| L2079 | Zm00001d047457 | PIP5K candidate |
| L2079 | Zm00001d029211 | PIP5K paralogue |
| L2079 | Zm00001d029209 | PIP5K paralogue |
| L2079 | Zm00001d004955 | PIP5K paralogue |
| L2125 | Zm00001d015175 | Phosphatidylinositol/phosphatidylcholine transfer protein |
| L2125 | Zm00001 d016601 | Phosphatidylinositol/phosphatidylcholine transfer protein |
| L2122 | Zm00001 d052022 | Plasma membrane ATPase candidate |
| L2122 | Zm00001 d045122 | Plasma membrane ATPase paralogue |
| L2124 | Zm00001 d014157 | NAD(P)H dehydrogenase / Flavoprotein wrbA |
| L2124 | Zm00001 d043249 | Minor allergen / NAD(P)H dehydrogenase |
| L2124 | Zm00001 d012607 | Minor allergen / NAD(P)H dehydroqenase |
| L2120 | Zm00001 d024533 | Putative DEAD-box ATP-dependent RNA helicase family protein |
| L2121 | Zm00001 d007712 | p-loop nucleoside triphosphate hydrolase superfamily protein with Calponin Homology domain |
| L2121 | Zm00001 d048673 | p-loop nucleoside triphosphate hydrolase superfamily protein with Calponin Homoloqy domain |
| L2123 | Zm00001 d037643 | Phospholipase D ZmPLD3 |
| L2123 | Zm00001 d005770 | Phospholipase D ZmPLD8 |

The haploid inducer plant line is a monocotyledon or dicotyledon plant line, preferably a monocotyledon plant line. According to an embodiment, the monocotyledonous plant line is a maize line.

### Methods for producing a haploid inducer plant line

For producing a haploid inducer plant line a method is provided which comprises:
(a) mutated gene leads to the modulation modulating of the expression of a gene in a plant, wherein the modulation of expression of said gene results in a loss of function of the gene and causes defective sperm cell function; or
(b) mutated gene leads to the modulating of the expression of a gene in a plant, wherein the modulation of expression of said gene results in a loss of function of the gene and promotes fertilization of central cell;
   or
(c) modulating the expression of *GEX2* and/or *KPL* gene in a plant, wherein the modulation of expression of *GEX2* and/or *KPL* gene results in a loss of function of the gene(s);
whereby said plant exhibit haploid induction activity.

In some aspects the method producing a haploid inducer plant line comprises a mutated GEX2 and/or KPL gene that promotes fecundation of central cell.

The invention also relates to a method for producing a haploid inducer plant line according to the invention, comprising:
(c) modulating the expression of a first gene in a plant, wherein the modulation of expression of said first gene results in a loss of function of the gene and causes defective sperm cell function; and
   modulating the expression of a second gene in a plant, wherein the modulation of expression of said second gene results in a loss of function of the gene and promotes fecundation of central cell;
   with the proviso that if the first gene is the gene *MATRILINEAL*/*NOT LIKE DAD*/*ZmPHOSPHOLlPASE-A1,* and the second gene is *DMP* gene, then expression of a third gene is modulated to result in a loss of function of the gene and cause defective sperm cell function or promotion of fecundation of central cell; or
(b) modulating the expression of *GEX2* and/or *KPL* gene in a plant, wherein the modulation of expression of *GEX2* and/or *KPL* gene results in a loss of function of the gene(s);
whereby said plant exhibit haploid induction activity.

Modulating the expression of said gene(s) comprises performing a technique selected from the group consisting of mutation, gene silencing, gene suppression, gene down-regulation, gene alteration, gene knock-down, RNA interference (RNAi), antisense, microRNAs, genetic transformation with a transgene, single and multiple gene conversion, gene transfer, genome editing tools including but not limited to meganucleases (MNs), zinc finger nucleases (ZFNs), transcription activator-like effector nucleases (TALENs), RNA-guided nucleases (RGNs), clustered regularly interspaced short palindromic repeat (CRISPR) and CRISPR-associated nucleases Cas9, SP Cas9, CasX, CasY, Cas12 (Cpf1), Cas14, and variants, and targetrons. The modulation of the expression of the gene can lead directly or indirectly to one of the two haploid causal pathway : "causing defective sperm cell function" or "promoting fertilization of central cell".

According to an embodiment, the method involves transforming a plant cell with at least said first and second mutated genes as defined above, or with at least mutated GEX2 and/or KPL gene, and regenerating a transformed plant from the transformed cell, thereby obtaining a haploid induction or enhancement of haploid induction by pollen of the transformed plant.

According to an embodiment, the method involves mutating at least said first and second genes as defined above, or mutating at least said GEX2 and/or KPL gene of a plant using a gene editing method. Preferably, the mutation is a loss-of-function or knock-down mutation.

According to an embodiment, the method involves (i) transforming a plant cell with one of said first or second mutated gene as defined above, and (ii) mutating the other one of said first or second gene as defined above, preferably by a loss-of-function or knock-down mutation.

Accordingly, the method may then comprise:
(a) transforming a plant cell with at least said first and/or second mutated genes, or with at least mutated GEX2 and/or KPL gene and regenerating a transformed plant from the transformed cell, thereby obtaining a haploid induction or enhancement of haploid induction by pollen of the transformed plant; and/or
(b) mutating at least said first and/or second genes, or mutating at least said GEX2 and/or KPL gene of a plant using a gene editing method.

Said process and said plant transformation involve, and are not limited to, introducing a DNA or RNA construct into a plant or host cell. "Introducing" or "administering" is intended to mean presenting to the plant a DNA or RNA construct. Processes for introducing polynucleotides into plants are known in the art including, and not limited to, stable transformation methods and transient transformation methods.

"Transformation" refers to the transfer of a nucleic acid fragment into the genome of a host organism, resulting in genetically stable inheritance. Host organisms containing the transformed nucleic acid fragments are referred to as "transgenic" organisms.

"Host cell" refers the cell into which transformation of the DNA construct takes place and may include a yeast cell, a bacterial cell, and a plant cell. Examples of methods of plant transformation include Agrobacterium-mediated transformation (De Blaere et al., 1987, Meth. Enzymol. 143:277), particle-accelerated or "gene gun" transformation technology (Klein et al., 1987, Nature (London) 327:70-73; U.S. Patent No. 4,945,050) and protoplast transformation by electroporation or the use of chemicals such as PEG, among others.

"Stable transformation" is intended to mean that the polynucleotide introduced into a plant integrates into the genome of the plant and is capable of being inherited by the progeny thereof. "Transient transformation" or "transient expression" is intended to mean that the polynucleotide is introduced into the plant and does not integrate into the genome of the plant.

A "vector" is a construct that includes a replication system and sequences that are capable of transcription and translation of a polypeptide-encoding sequence in a given host cell. A number of vectors suitable for stable transfection of plant cells or for the establishment of transgenic plants have been described in, e.g., Pouwels et al., Cloning Vectors: A Laboratory Manual, 1985, supp. 1987; Weissbach and Weissbach, Methods for Plant Molecular Biology, Academic Press, 1989; and Flevin et al., Plant Molecular Biology Manual, Kluwer Academic Publishers, 1990. Typically, plant expression vectors include, for example, one or more cloned plant sequences under the transcriptional control of 5' and 3' regulatory sequences and a dominant selectable marker. 5' and 3' regulatory sequences comprise but are not limited to a promoter regulatory region (e.g., a regulatory region controlling inducible or constitutive, environmentally or developmentally-regulated, or cell-or tissue-specific expression), transcription initiation start site, ribosome binding site, RNA processing signal, transcription termination site, and polyadenylation signal. In particular, the vectors according to the invention can be chosen from vectors pSB11 (*Ishida et al.,* (1996).), pBIOS898 (Biogemma, for sense construct), pBIOS895 (Biogemma, for RNAi construct), pBb7m34GW, pBb7m42GW7 (Karimi *et al.,* 2012), pDONR221 (Invitrogen).

By "plasmid", it is herein meant a double-stranded circular DNA. The plasmid may include a marker gene enabling to select the cells comprising said plasmid, an origin of replication to allow the cell to replicate the plasmid and/or a multiple cloning site allowing the insertion of a DNA fragment, in particular the polynucleotide according to the invention. In particular, said plasmid is chosen from L1457, L1465, L1478, L1482, L1479, L1483, L1542, L1543, L1540, L1541, as described in WO 2016/177887.

The vector preferably comprises an expression cassette of the polynucleotide of interest, i.e. a nucleic acid comprising to the polynucleotide of interest placed under the control of at least one expression signal allowing its expression.

The expression signal is particularly selected among a promoter, a terminator, an enhancer and their combinations. Suitable promoters, terminators and enhancers are well-known by the skilled person. In particular, the promoter may be a constitutive promoter selected in the group consisting of the rice actin promoter (Act1) and intron (McElroy et al., 1990, Plant Cell, 2 :163-171), the ubiquitin promoter of maize (Christensen et al., 1996, Transgenic. Res., 5 :213) or the CsVMV promoter (Verdaguer et al., 1998, Plant Mol Biol. 6:1129-39) and FAD₂ intron (patent application WO 2006/003186 from Biogemma). Other examples of constitutive promoters useful for expression include the 35S promoter, the 19S promoter (Kay et al., 1987, Science, 236:1299-1302), or the pCRV promoter (Depigny-This et al., 1992, Plant Molecular Biology, 20 :467-479). In a preferred embodiment, said construct is under the control of a constitutive promoter. In a most preferred embodiment, said construct is an RNAi construct, under the control of a constitutive promoter. Other suitable promoters could be used. It could be a tissue-specific promoter, for example a pollen-specific promoter, a promoter active in both anther and pollen, or an inducible promoter.

A terminator may be selected in the group consisting of the Nos terminator corresponding to the region in the non coding 3' region of the nopaline synthase gene of the Ti-plasmid of *Agrobacterium tumefaciens* nopaline strain (Depicker et al. 1992) or the AtSac66 terminator. Others possible terminators are the polyA 35S terminator of the cauliflower mosaic virus (CaMV), described in the article of Franck *et al.,* (1980) or the histone terminator (EP 0 633 317).

Any other element like introns, enhancers, transit peptides, etc... may be comprised in the expression cassette. Introns and enhancers may be used to improve the expression of the polynucleotide of interest.

Suitable methods of introducing polynucleotides into plant cells are known by the man skilled in the art and include Agrobacterium- mediated transformation (U.S. Patent Nos. 5,563,055 and 5,981,840, and Ishida et al*.,* (1996) High efficiency transformation of maize (Zea mays L.) mediated by Agrobacterium tumefaciens (Nat Biotechnol. 1996 Jun;14(6):745-50.), particle bombardment (Gordon-Kamm et al., 1990, Plant Cell 2:603-618) and electroporation of protoplasts (Rhodes et al., 1988, Science 240:204-207).

Methods are known in the art for the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853. Briefly, the polynucleotide according to the invention can be contained in a transfer cassette flanked by two non-identical recombination sites. An appropriate recombinase is needed for the integration of the transfer cassette fragment at the target site. Other methods without recombinase have also been described in WO 90/11354 but also in WO 96/14408. Said methods start from the observation that Double Strand Break (DSB) enhances the probability of homologous recombination at a given position. Said methods have been improved by the development of Meganucleases, Zn finger nuclease and other tools like TALENs and CRISPR/Cas9 system that have been developed to obtain targeted DSB.

The cells that have been transformed may be grown into plants in accordance with conventional ways. See, for example, McCormick et al. (1986) Plant Cell Reports 5:81-84.

These tools can be used for "gene editing". The terms "gene editing" cover targeted mutations, theses mutations can be random mutation or directed mutations, by targeted is intend that the localization of the mutation is chosen. In particular, by the previously described homologous recombination system, a nucleotide exchange can be done to induce targeted mutations. Other methods for "gene editing" include the use of a DSB and a repair template to induce a specific nucleotide exchange during DNA repair. The CRISPR/Cas9 system is one of the specific methods of "gene editing" whereas the Cas9 protein and an ARN are used for obtaining a targeted DSB. Alternatively a simple DSB (double strand break) without repair template can be done on a targeted sequence to induce random mutations at this site. These mutations should be short insertions or deletions based on NHEJ (near Homologous End Joining) or MMEJ (microhomology mediated end joining).

RNA interference (RNAi) can also be induced in the plant to repress expression of a first gene, which causes defective sperm cell function when repressed, and to repress expression of a second gene, which promotes fertilization of central cell when repressed. By "RNA interference" is meant the biological process in which RNA molecules inhibit gene expression, typically endogenous gene expression by causing the destruction of specific mRNA molecules.

According to this aspect, a plant is transformed with a DNA or RNA molecule interfering with expression of said first gene, and a DNA or RNA molecule interfering with expression of said second gene, or a DNA or RNA molecule interfering with expression of *GEX2* and/or *KPL* gene.

The RNA molecule can be a small interfering RNA (siRNA) molecule comprising 18-25 nucleotides of said first gene, and a siRNA molecule comprising 18-25 nucleotides of said second gene, as defined above, or administering to a plant at least one siRNA molecule comprising 18-25 nucleotides of GEX2 and/or KPL gene. A siRNA typically comprises 18-25, e.g. 19-23 or preferably 19-21 contiguous nucleotides of the target gene.

The RNA molecule can be a double stranded RNA (dsRNA). As used herein, a dsRNA molecule is a RNA molecule comprising at least one annealed, double stranded region. In certain aspects, the double stranded region comprises two separate RNA strands annealed together. In certain aspects, the double stranded region comprises one RNA strand annealed to itself, for example, as can be formed when a single RNA strand contains an inversely repeated sequences with a spacer in between ("hairpin RNA"). Expression of hairpin RNA in cells is typically accomplished by delivery of a DNA construct by plasmids or through viral or bacterial vectors, which is transcribed in a hairpin RNA.

A dsRNA molecule comprises a nucleic acid sequence complementary to about 21 to 2000 contiguous nucleotides of a target gene sequence discloses anywhere herein. For example, in certain aspects, an dsRNA molecule comprises a nucleic acid sequence complementary to about any of 21, 22, 23, 24, 25, 30, 100, 200, 300, 400, 500, 1000 to about any of 23, 24, 25, 30, 40, 50, 100, 200, 300, 400, 500, 1000, or 2000 contiguous nucleotides of a target gene sequence. For example, in certain aspects, a dsRNA molecule comprises a nucleic acid sequence complementary to about 100 to 1000 or about 200 to 1000 contiguous nucleotides of a target gene sequence. For example, in certain aspects, a dsRNA molecule comprises a nucleic acid sequence complementary to about 100 to 1000 or about 200 to 1000 contiguous nucleotides of the protein coding region of a target gene sequence. For example, in certain aspects, a dsRNA molecule comprises a nucleic acid sequence complementary to about 100 to 1000 or about 200 to 1000 contiguous nucleotides of the 5' UTR region or the 3' UTR region of a target gene sequence.

### Methods for producing a haploid plant or doubled haploid plant

For producing a haploid plant, it is provided a method that may comprise:
(a) pollinating a female plant with pollen from a haploid inducer plant line as defined herein; and
(b) screening the progeny of pollinated female plant for selecting haploid plants.

More specifically, said method comprises a) crossing a haploid inducer plant line as defined herein, used as pollinator, with a female plant; and (b) screening the progeny of the cross for selecting haploid plants.

A method for producing a haploid plant may also comprise:
(a) exposing pollen from a haploid inducer plant line comprising a mutated gene promoting fertilization of central cell to an agent causing chromosome fragmentation;
(b) pollinating a female plant with said pollen exposed in (a) to the agent causing chromosome fragmentation;
(c) screening the progeny of pollinated female plant for selecting haploid plants.

According to an embodiment, in said method, the gene which mutation promotes fertilization of central cell is selected from the group consisting of *DMP, KPL,* and *GEX2.*

A method for producing a haploid plant may also comprise:
(a) pollinating a female plant simultaneously, separately or successively with (i) pollen from a first haploid inducer plant line comprising a mutated gene causing sperm cell to contain defective chromosomes, and (ii) pollen from a second haploid inducer plant line comprising a mutated gene causing defective double fertilization;
(b) screening the progeny of the pollinated female plant for selecting haploid plants produced by heterofertilization.

Preferably, said female plant is pollinated successively with pollen from a first haploid inducer plant line comprising a mutated gene causing sperm cell to contain defective chromosomes, and then with (ii) pollen from a second haploid inducer plant line comprising a mutated gene causing defective double fertilization.

According to an embodiment of said method, the mutated gene causing sperm cell to contain defective chromosomes and the mutated gene causing defective double fertilization are not simultaneously *MATRILlNEAL*/*NOT LIKE DAD*/*ZmPHOSPHOLIPASE-A1* gene and *DMP* gene, respectively.

According to an embodiment of the methods for producing a haploid plant, the gene which mutation causes sperm cell to contain defective chromosomes is selected from the group consisting of *NLD,* a gene encoding Phosphatidylinositol 4-phosphate 5-kinase, a gene encoding Phosphatidylinositol/phosphatidylcholine transfer protein, a gene encoding Phospholipase D, a gene which is Zm00001d042182 or an orthologous thereof, a gene encoding an Oleosin, a gene encoding a Nucleic acid-binding OB-fold-like protein, and a gene encoding beat-glucanase. Preferably, the first gene which mutation causes defective sperm cell function is selected from the group consisting of *NLD* gene, a gene encoding Phosphatidylinositol 4-phosphate 5-kinase, and a gene encoding Phosphatidylinositol/phosphatidylcholine transfer protein.

According to some embodiments, the second gene which mutation promotes fertilization of central cell is selected from the group consisting of *DMP, KPL,* and *GEX2* genes.

According to an advantageous aspect of the methods, the pollen from the haploid inducer plant line contains a CRISPR-Cas9 cassette designed to target one or more gene(s) of interest. The target gene is typically a gene thought to be associated with a desired agronomic trait.

The haploid plant produced by any one of the methods described herein may be used in a method for generating a doubled haploid plant which comprises inducing chromosome doubling in the haploid plant.

To that end, the haploid genetic material is typically treated with one or more mitotic arrest agents to allow the haploid (1n) chromosome complement in one or more cells to produce homolog pairs. After the chemical treatment procedure, the chromosome doubling chemical(s) are removed. A chromosome doubling agent such as colchicine or trifluralin, may be used to form doubled haploid homozygous inbred lines.

As used herein, a plant referred to as "doubled haploid" is developed by doubling the haploid set of chromosomes. A plant or seed that is obtained from a doubled haploid plant that is selfed to any number of generations may still be identified as a doubled haploid plant. A doubled haploid plant is considered a homozygous plant. A plant is considered to be doubled haploid if it is fertile, even if the entire vegetative part of the plant does not consist of the cells with the doubled set of chromosomes, that is, a plant will be considered doubled haploid if it contains viable gametes, even if it is chimeric.

The term "comprising," is inclusive or open-ended and does not exclude additional, unrecited elements and/or method steps. "Comprising" is a term of art that means that the named elements and/or steps are present, but that other elements and/or steps can be added and still fall within the scope of the relevant subject matter. Accordingly, in certain aspects, the disclosure of a method or product comprising elements and/or steps also discloses a method or product consisting of these elements and/or steps.

The tem "consisting of" excludes any element, step, or ingredient not specifically recited.

The invention will be further illustrated by the following Figures and Examples.

### FIGURES

**Figure 1****.** Identification of putative maternal haploid plants in *A. Thaliana* using glabra phenotype (no trichomes), haploid plants are sterile (absence of silique) with reduced organ size. Plants are offspring of Atgl plant crossed by Atdmp8/9^{c} (*Atdmp8*/*9* mutant used as positive control of haploid induction) (A), Atgex 2-2 (B), Atkpl-1 (C) and Atkpl-2 (D).
**Figure 2****.** Confirmation of polyploidy level by flow cytometry of progeny of *figure* mutants (Dolezel et al., 2007, Nat Protoc 2, 2233-2244; .DeLaat et al., 1987, Plant Breeding, 99: 303-307). Upper graph is a diploid plant and lower graph a putative haploid plant. Plants of figure 1, are offspring from the crosses *gl* x dmp8/9^{c} (*Atdmp8*/*9* mutant used as positive control of haploid induction) (A); *gl* x *kpl-1* (C) and *gl* x *kpl-2*(D)*.* Arrows indicate haploid peaks.

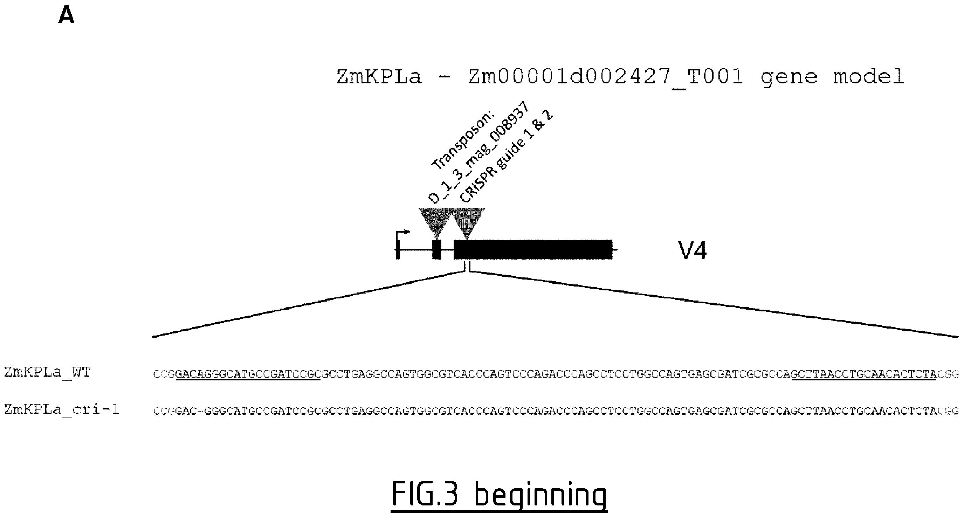

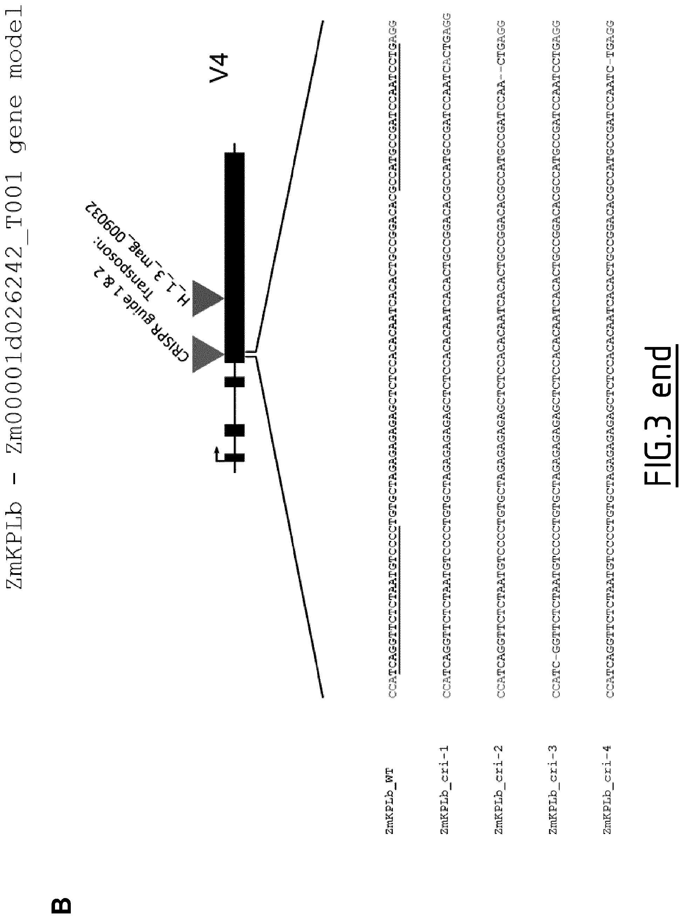

**Figure 3****.** Description of knockout mutations in *ZmKpla* (A) *and ZmKplb* (B) ; Wt sequences originates *from* Zm00001d002427 (AGP V4) for ZmKpla and Zm00001d026242 (AGP V4) for ZmKplb . An insertion mutant has been identified for Zmkpla and the insertion point is identified D 1-3_mag 008937, a CRISPR/Cas9 mutant has been generated using L2126 CRISPR vector (underlined sequence on the wt sequence corresponds to guide sequence) *Zmk*pla cri-1 (SEQ ID NO:16). (A) ; An insertion mutant has been identified for Zmkplb and the insertion point is identified H 1-3_mag 009032, and four CRISPR/Cas9 mutants have been generated using L2126 vector, *Zmkp*/b cri-1 to Zmkplb cri-4 (B) (SEQ ID NO:17 to SEQ ID NO:20).
**Figure 4****.** Measurement of the induction rate for single and double mutants of ZmKpla, ZmKplb and ZmNLD (nld -/-), ZmKpla mutants (kpla -/-) and ZmKplb mutants (kplb -/-) are transposable insertion (Mu) insertion mutants, mutation described in figure 3.
**Figure 5****.** Description of CRISPR/Cas9 generated mutants for Phosphatidylinositol/phosphatidylcholine transfer protein:Zm00001d015175 (A) and Zm00001d016601 (B) using L2125 CRISPR vector; for Zm00001d015175, Wt sequences originates from A188 and two CRISPR/Cas9 mutant are described C5175_cri-2 (SEQ ID NO:21) and C5175_cri-3 (SEQ ID NO:22); for Zm00001d016601 Wt sequences originates from P6601 and four CRISPR/Cas9 mutant are described P6601_cri-8 (SEQ ID NO:23); P6601_cri-1 (SEQ ID NO:24); P6601_cri-7 (SEQ ID NO:25) and P6601_cri-9 (SEQ ID NO:26).
**Figure 6****.** Identification of putative maternal haploid plant using glossy phenotype and organ size: Pollen from Zm00001 d015175/Zm00001d016601 double heterozygous plant has been used on glossy plants, plant phenotypically identified as haploid has been generated (A). Haploidy has been confirmed by flowcytometry (B). Arrows indicate haploid peaks.

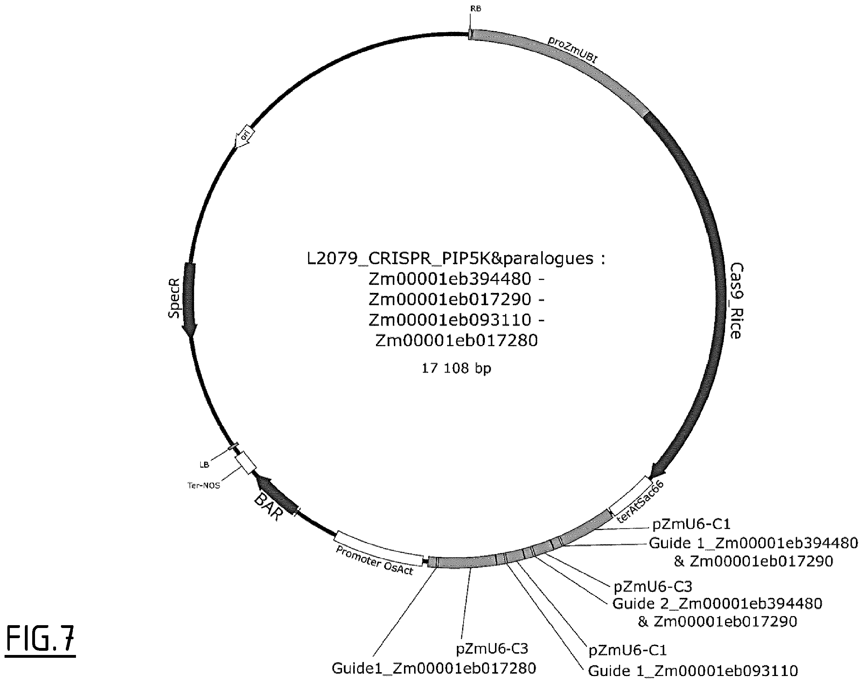

**Figure 7****.** Plasmid map for CRISPR vector L2079 used to knock-out the PIP5K genes: Zm00001eb394480 - Zm00001 eb017290 - Zm00001 eb093110 - Zm00001eb017280.

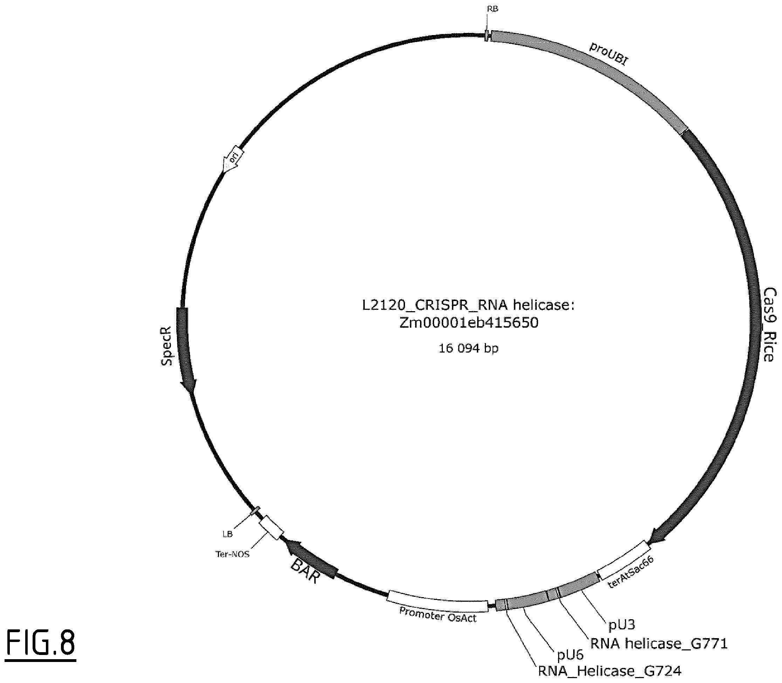

**Figure 8****.** Plasmid map for CRISPR vector L2120 used to knock-out RNA helicase gene: Zm00001eb415650.

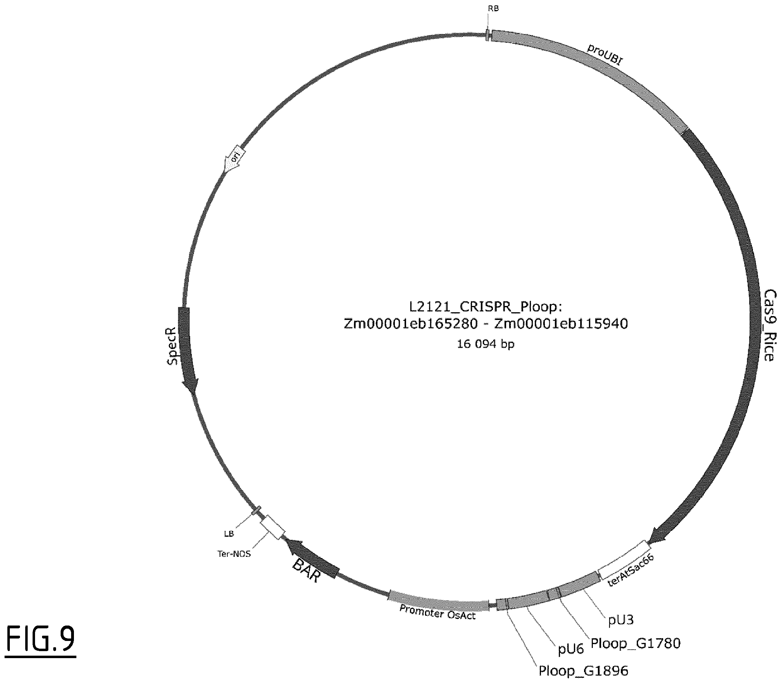

**Figure 9****.** Plasmid map for CRISPR vector L2121 used to knock-out _Ploop gene: Zm00001eb165280 - Zm00001eb115940.

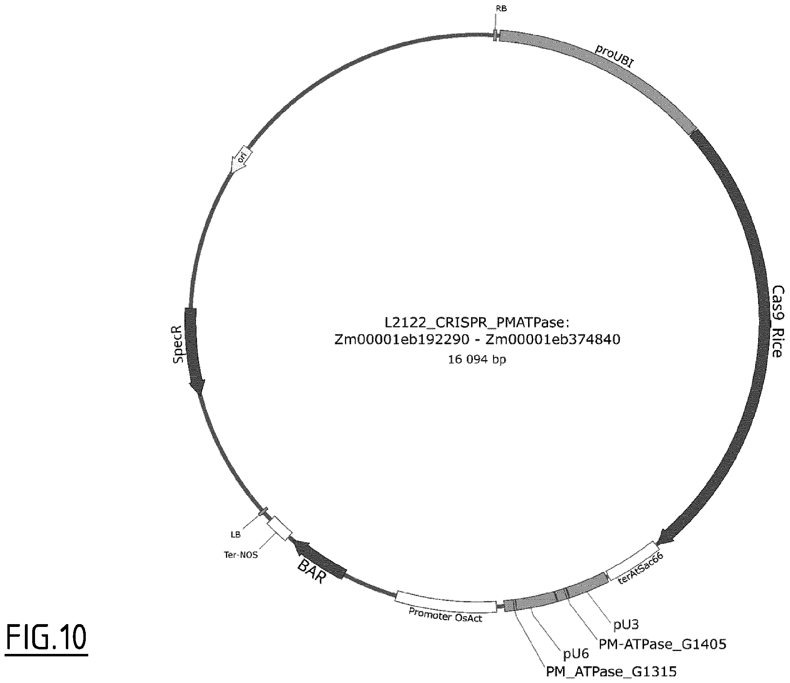

**Figure 10****.** Plasmid map for CRISPR vector L2122 used to knock-out the PMATPase: genes Zm00001eb192290 - Zm00001eb374840.

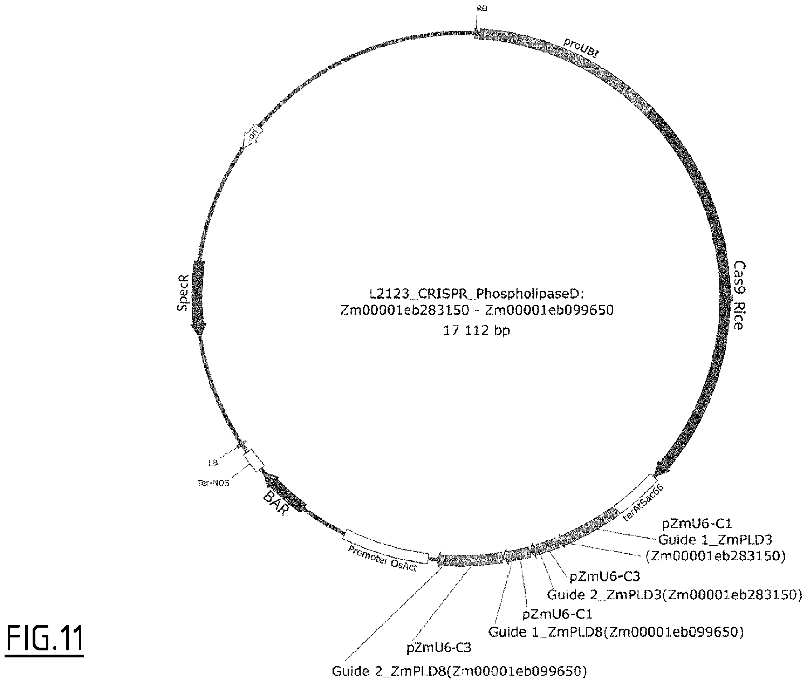

**Figure 11****.** Plasmid map for CRISPR vector L2123 used to knock-out the phospholipase D genes: Zm00001 eb283150 - Zm00001 eb099650.

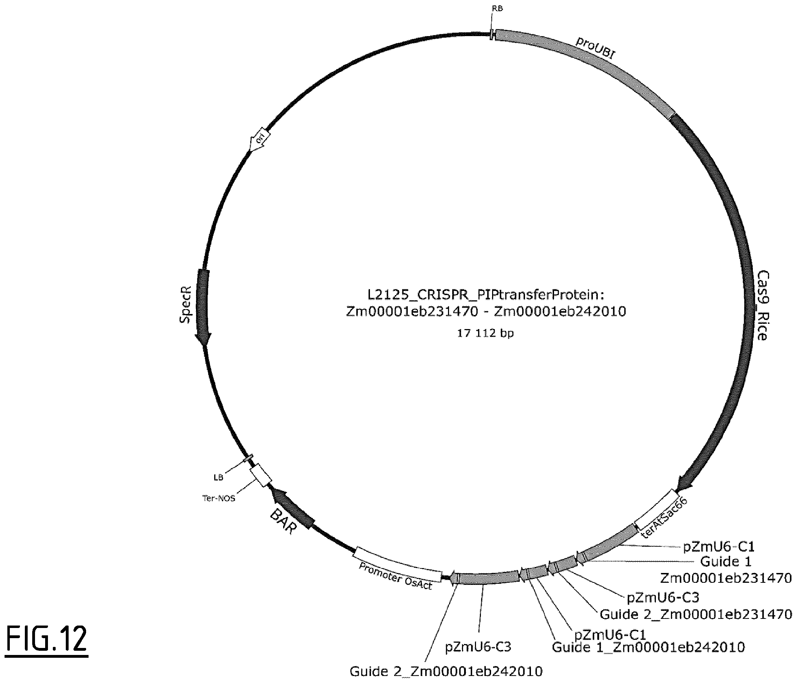

**Figure 12****.** Plasmid map or CRISPR vector L2125 used to knock-out the PIPtransferProtein genes: Zm00001eb231470 - Zm00001eb242010.

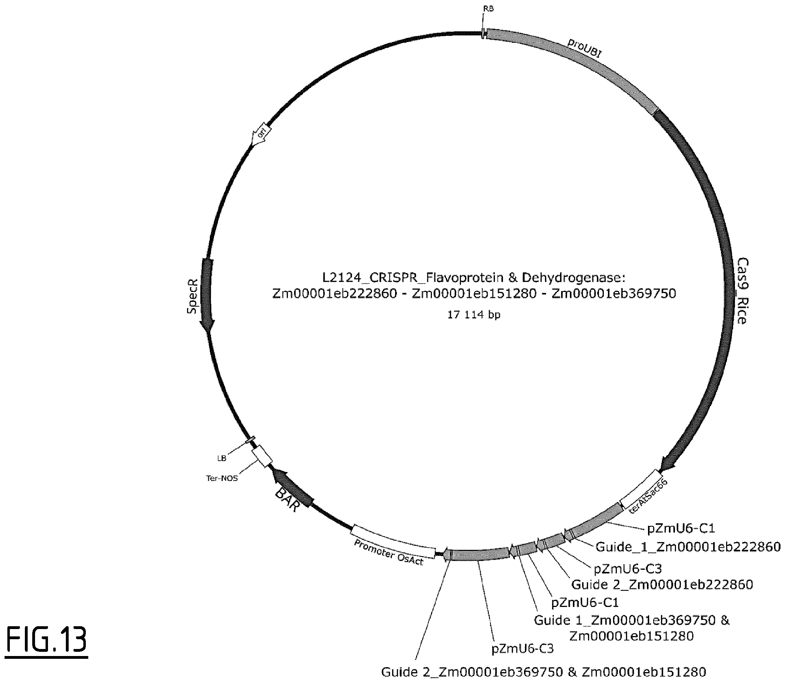

**Figure 13****.** Plasmid map for CRISPR vector L2124 used to knock-out the Flavoprotein & Dehydrogenase genes: Zm00001eb222860 - Zm00001eb151280 - Zm00001eb369750.

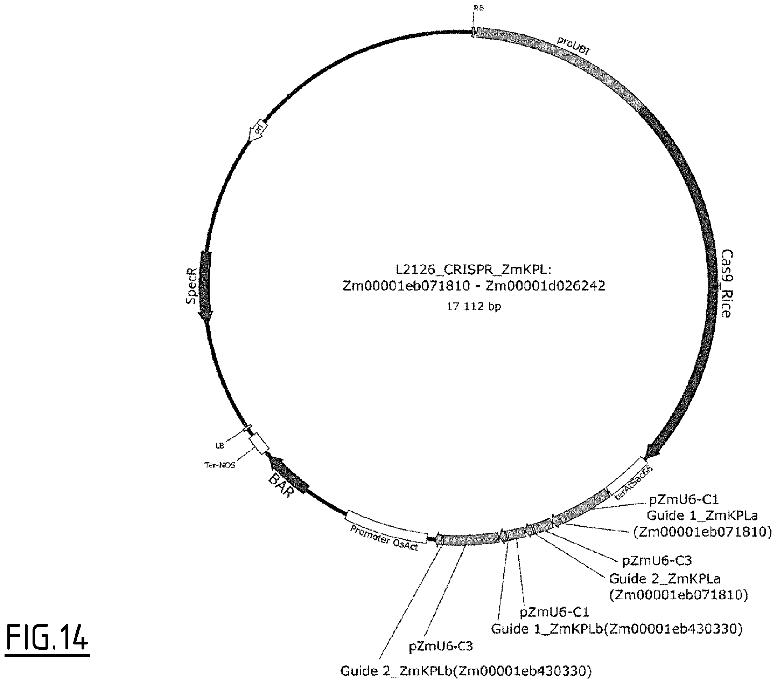

**Figure 14****.** Plasmid map for CRISPR vector L2126 used to knock-out the ZmKPLa and ZmKPLb genes: Zm00001eb071810 - Zm00001d026242.

### EXAMPLES

### Example 1

*Arabidopsis* male gametophytic genes, mutations of which lead to single fertilization events similarly to what was reported for *Atdmp8,9* mutations. *AtGEX2* (*GAMETE EXPRESSED 2, AT5G49150)* and *AtKPL* (KOKOPELLI, AT5G63720) were selected (Maruyama et al., 2013, Developmental Cell, 25, 317-323; Mori et al., 2014, Current Biology, 24, 170-175; Ron et al., 2010, Genes Dev., 24, 1010-1021; Xiong et al., 2021, The Plant Cell, 33, 1151-1160).

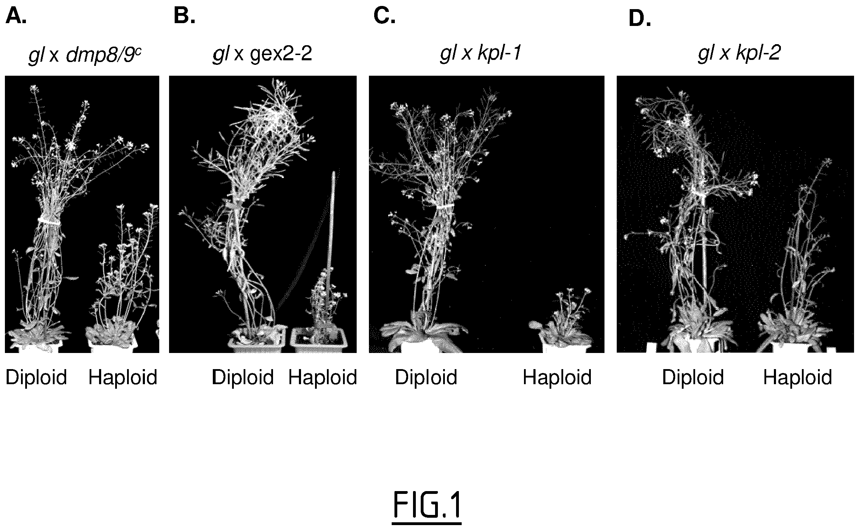

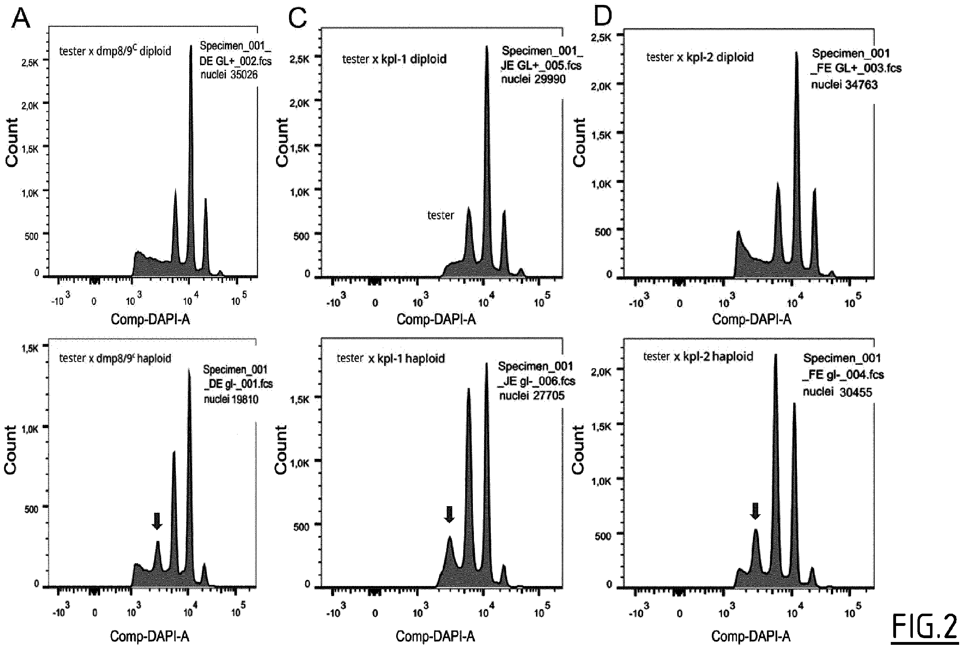

In order to detect maternal haploid induction, a female tester line having the recessive phenotypic marker absence of trichomes (glabra, *gl*) was used. Haploid inducing capacity of a given mutant was evaluated by adding mutant pollen on pistils of the *gl* tester line, and using the following pipeline: (1) The offsprings were germinated and screened for absence of trichomes indicating putative maternal haploid seedlings; (2) Putative haploid seedlings were then checked for typical haploid phenotype: sterility (absence of silique) and smaller organs **(****Figure 1****);** and (3) Haploidy was assessed by flow cytometry on leaves **(****Figure 2****).**

**Table 4: Haploid induction rate of different genotypes**

| **Genotype** | **Plants screened** | **Haploid** | **haploid induction rate (HIR)** |
|---|---|---|---|
| col0 (WT) | 937 | 0 | 0,000% |
| ler (WT) | 734 | 0 | 0,000% |
| WS4 (WT) | 813 | 0 | 0,000% |
| *Atgex2-1* | 174 | 0 | 0,000% |
| *Atgex2-2* | 966 | 1 | 0,103% |
| *Atkpl-1* | 747 | 2 | 0,267% |
| *Atkpl-2* | 1026 | 1 | 0,097% |
| *Atdmp8*/*9* | 326 | 6 | 1,807% |

For both *AtGEX2* and *AtKPL,* two independent mutant lines were selected, and the *Atdmp8*/*9* mutant was used as positive control of haploid induction. Using the pipeline described above, the previously reported haploid induction capacity of *Atdmp8,9* mutant was confirmed since we identified 6 haploids out of 326 plants screened. Using *Atgex2-2* mutants, 1 haploid among 1140 plants was identified. Haploid induction capacity was also demonstrated for *Atkpl* mutant with 3 haploids among 1773 plants. No haploid plants were detected in the wild-type accessions (0/2484). **Figure 1** shows haploid plants in comparison with diploid siblings. Confirmation of haploidy was successfully achieved using flow cytometry (**Figure 2****, C and D** for Atkpl mutants).

### Example 2

Since Atk*pl* mutant is able to induce maternal haploid embryos in *Arabidopsis thaliana,* maize orthologous genes were investigated. Using information from the maize B73 genome sequence (AGP v5), P-blast search identified two maize genes: Zm00001eb071810 (ZmKPLa) and Zm00001eb430330 (ZmKPLb). Loss of function mutants were identified in a transposable element (Mu) insertion collection, as well as generated using CRISPR/Cas9 strategy as described in (Doll et al., 2019, Plant Cell Rep 38: 487-501) (**Figures 3**).

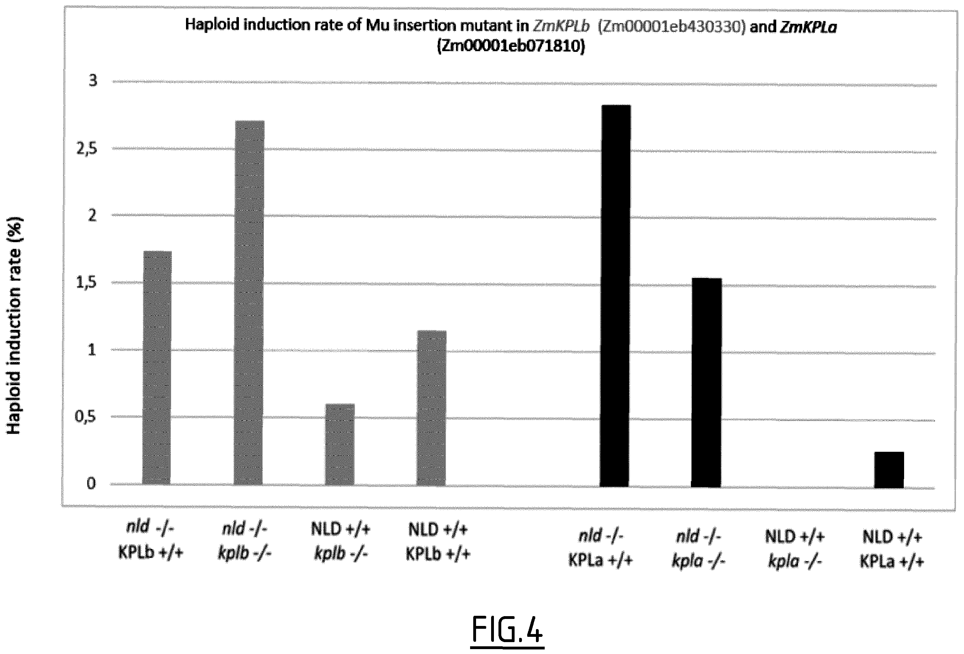

Haploid induction capacity for the two Mu Mutants were tested using a glossy tester line as reported previously (Gilles et al., 2017, EMBO J 36: 707-717). Haploid plants were detected in the progeny of *Zmkplb* single mutant (kplb -/-), and not in the *Zmkpla* single mutant (kpla -/-) (**Figure 4**). Haploid induction was also detected in the CRISPR mutant ZmKPLb_cri-1 (in A188 genetic background) with a haploid induction rate of 1,43% (5 haploids seedling out of 349).

*zmkpl* single Mu insertion mutants were combined with the *nld* mutation. The *nld*/*zmkplb* double mutant has a higher haploid induction rate (2.7%) as compared to the *nld* single mutant (1.7%) or to the *zmkplb* single mutant (0.6%) (**Figure 4**).

Mutants obtained by CRISPR/cas9 strategy are also tested for haploid induction and double mutants are also produced.

### Example 3

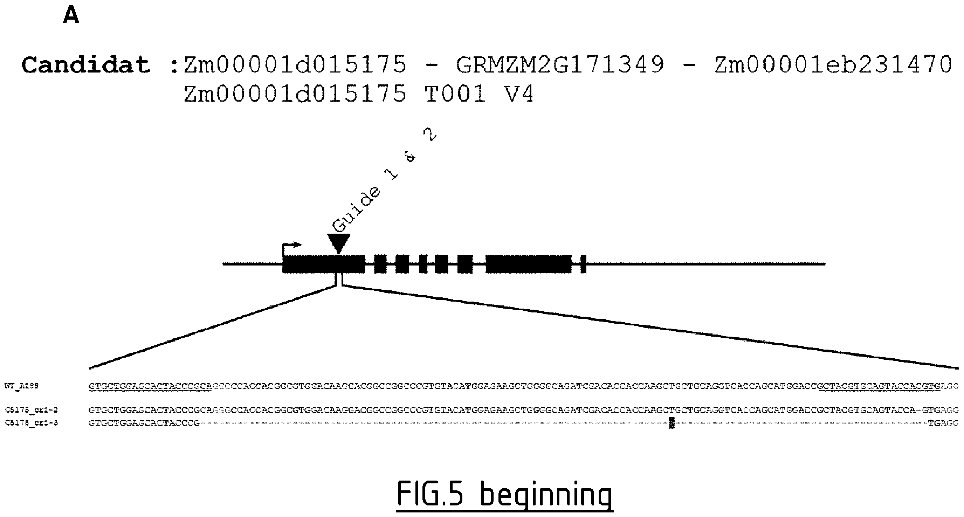

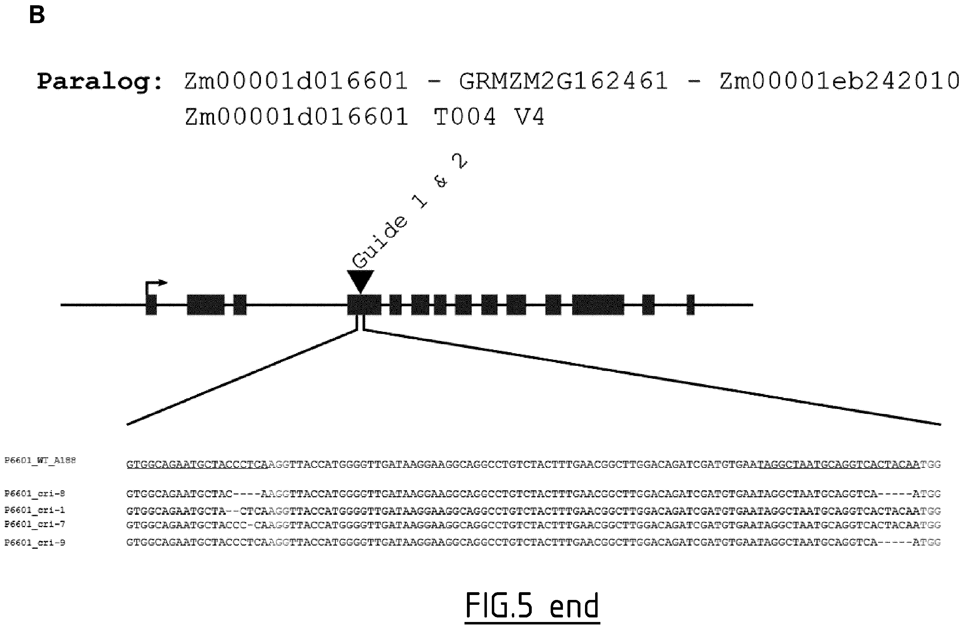

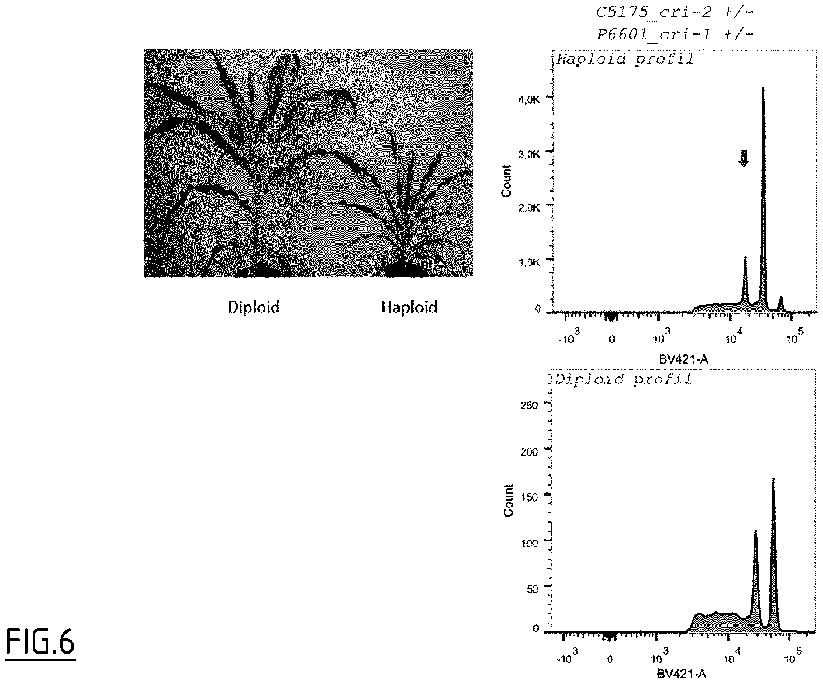

The gene Zm00001d015175 was identified as putative interactor of NOT-LIKE-DAD protein using immunoprecipitation. This gene is annotated as coding for "phosphatidylinositol/phosphatidylcholine transfer protein" and has a paralogous gene (Zm00001d016601) which is also highly expressed in pollen grain. CRISPR/Cas9 strategy, as described in (Doll et al., 2019, Plant Cell Rep 38: 487-501), was applied to generate mutants in both genes (**Figures 5**). Screening among 400 offspring of selfed double heterozygous mutant plants for Zm00001 d015175 or Zm00001d016601 did not allow to recover any homozygous mutant plants, indicating defects in either gamete formation or lethality of homozygous plants. Using pollen from Zm00001d015175/Zm00001 d016601 double heterozygous plants on glossy tester plants allow to identify 2 haploid plants out of 374 plants screened (**Figures 6**). The corresponding A188 wild-type plant do not induce haploid embryos (0/5500). Haploid were confirmed using flow cytometry (Figure 6, B).

### Example 4: Combination of mutants defective in double fertilization with irradiation

Plants mutated in genes promoting fertilization of central cell (DMP or GEX2 or KPL) is combined with physical agent causing chromosome fragmentation, such as gamma- or X-ray irradiation. Pollen from dmp or gex2 or kpl mutant is directly irradiated with gamma- or X-ray sources and then used to pollinate whatever female plants to generate maternal haploids embryos. Alternatively, a first pollination with non-irradiated pollen from dmp or gex2 or kpl is done in order to induce correct endosperm development, and then a second pollination is done with irradiated pollen. This situation favor the development of seeds with correct endosperm development and haploid embryo, creating *in vivo* haploid induction.

### Example 5: Creation of defective sperm cells for haploid induction and combination with mutants defective in double fertilization

Instead of using loss of function mutants in gene(s) or radiation treatments to generate defective sperm cell function, CRISPR/cas9 strategy is used to specifically impaired sperm cells. The CRISPR/kill system, which is able to induce multiple double strand breaks in repetitive DNA, was shown to be efficient to specifically block organogenesis (Schindele et al., 2022, Nat Commun 13: 1502). Thus, using sperm cell type-specific promoters, such as the one from MALE-GAMETE-SPECIFIC HISTONE H3 (MGH3/AT1G19890) or DUO POLLEN 1 (DUO1/ AT3G60460) in Arabidopsis, to drive the expression of the CRISPR-Kill cassette specifically in pollen generative cell and/or in sperm cells, defective sperm cells is generated. The pollen from sperm cell specific CRISPR-Kill plants is then used on whatever female genotype to induce haploid embryos. Embryo rescue is used in the case of endosperm development failure. To overcome this endosperm failure, combination of sperm cell specific CRISPR/kill system with *dmp* or *gex2or kplpollen* mutant is done. Either by doing a first pollination with *dmp* or *gex2* or *kpl* pollen follow by a second pollination with CRISPR/kill pollen, or in direct combination pollen with both *dmp* or *gex2 or kpl* mutation and CRISPR/kill cassette in the same pollen grain.

Similar strategy is done in egg cell, to induce paternal haploid embryo formation. In this case egg cell specific promoter such as the one from EGG CELL 1.1 gene (EC1.1/AT1G76750) is used to drive the expression of the CRISPR-Kill cassette specifically in egg cell. Pollination of those plants with wild-type pollen lead to formation of embryo with paternal genetic information, but the development of a correct endosperm to support seed growth.

## Claims

1. A haploid inducer plant line, wherein said haploid inducer plant line comprises at least
a) a first mutated gene causing defective sperm cell function;
b) a second mutated gene promoting fertilization of central cell;
with the proviso that if the first mutated gene is the gene *MATRILINEAL*/*NOT LIKE DAD*/*ZmPHOSPHOLIPASE-A1,* and the second mutated gene is *DMP* gene, then a third mutated gene is present causing defective sperm cell function or promoting fertilization of central cell.

2. The haploid inducer plant line according to claim 1, wherein the first gene which mutation causes defective sperm cell function is selected from the group consisting of *NOT LIKE DAD (NLD),* a gene encoding Phosphatidylinositol 4-phosphate 5-kinase, a gene encoding Phosphatidylinositol/phosphatidylcholine transfer protein, a gene encoding Phospholipase D, a gene which is Zm00001d042182 or an orthologous thereof, a gene encoding an Oleosin, a gene encoding a Nucleic acid-binding OB-fold-like protein, a gene encoding beta-glucanase, a gene encoding Putative DEAD-box ATP-dependent RNA helicase family protein, a gene encoding Plasma membrane ATPase, a gene encoding NAD(P)H dehydrogenase, and a gene encoding p-loop nucleoside triphosphate hydrolase superfamily protein with Calponin Homology domain.

3. The haploid inducer plant line according to claim 1 or 2, wherein the second gene which mutation promotes fertilization of central cell is selected from the group consisting of *DMP, KPL,* and *GEX2.*

4. A haploid inducer plant line, wherein said plant line comprises a mutated GEX2 and/or KPL gene that promotes fertilization of central cell.

5. The haploid inducer plant line according to any one of claims 1 to 4, wherein the plant is a monocotyledon or dicotyledon plant.

6. The haploid inducer plant line according to any one of claims 1 to 4, wherein the plant line is a maize line.

7. A method for producing a haploid inducer plant line as defined in any one of claims 1 to 6, wherein:
(a) Mutated gene leads to the modulating of the expression of a gene in a plant, wherein the modulation of expression of said gene results in a loss of function of the gene and causes defective sperm cell function; or
(b) Mutated gene leads to the modulating of the expression of a gene in a plant, wherein the modulation of expression of said gene results in a loss of function of the gene and promotes fertilization of central cell; or
(c) modulating the expression of *GEX2* and/or *KPL* gene in a plant, wherein the modulation of expression of *GEX2* and/or *KPL* gene results in a loss of function of the gene(s);
whereby said plant exhibit haploid induction activity.

8. The method of claim 7, which comprises:
(a) transforming a plant cell with at least said first and/or second mutated genes as defined in any one of claim 1 to 3, or with at least mutated GEX2 and/or KPL gene and regenerating a transformed plant from the transformed cell, thereby obtaining a haploid induction or enhancement of haploid induction by pollen of the transformed plant; and/or
(b) mutating at least said first and/or second genes as defined in any one of claim 1 to 3, or mutating at least said GEX2 and/or KPL gene of a plant using a gene editing method.

9. A method for producing a haploid plant, comprising:
(a) pollinating a female plant with pollen from a haploid inducer plant line as defined in any one of claims 1 to 6; and
(b) screening the progeny of pollinated female plant for selecting haploid plants.

10. A method for producing a haploid plant, comprising
(a) exposing pollen from a haploid inducer plant line comprising a mutated gene promoting fertilization of central cell to an agent causing chromosome fragmentation;
(b) pollinating a female plant with said pollen exposed in (a) to the agent causing chromosome fragmentation;
(c) screening the progeny of pollinated female plant for selecting haploid plants.

11. The method for producing a haploid plant according to claim 10, wherein the gene which mutation promotes fertilization of central cell is selected from the group consisting of *DMP, KPL,* and *GEX2.*

12. A method for producing a haploid plant, comprising:
(a) pollinating a female plant simultaneously, separately or successively with (i) pollen from a first haploid inducer plant line comprising a mutated gene causing sperm cell to contain defective chromosomes, and (ii) pollen from a second haploid inducer plant line comprising a mutated gene promoting fertilization of central cell;
(b) screening the progeny of the pollinated female plant for selecting haploid plants produced by heterofertilization.

13. The method for producing a haploid plant according to claim 12, wherein said female plant is pollinated successively with pollen from a first haploid inducer plant line comprising a mutated gene causing sperm cell to contain defective chromosomes, and then with (ii) pollen from a second haploid inducer plant line comprising a mutated gene promoting fertilization of central cell.

14. The method for producing a haploid plant according to claim 12 or 13, wherein the gene which mutation causes sperm cell to contain defective chromosomes is selected from the group consisting of *NLD,* a gene encoding Phosphatidylinositol 4-phosphate 5-kinase, a gene encoding Phosphatidylinositol/phosphatidylcholine transfer protein, a gene encoding Phospholipase D, a gene which is Zm00001d042182 or an orthologous thereof, a gene encoding an Oleosin, a gene encoding a Nucleic acid-binding OB-fold-like protein, a gene encoding beta-glucanase, a gene encoding Putative DEAD-box ATP-dependent RNA helicase family protein, a gene encoding Plasma membrane ATPase, a gene encoding NAD(P)H dehydrogenase, and a gene encoding p-loop nucleoside triphosphate hydrolase superfamily protein with Calponin Homology domain.

15. The method for producing a haploid plant according to any one of claims 12 to 14, wherein the gene which mutation promotes fertilization of central cell is selected from the group consisting of *DMP, KPL,* and *GEX2.*

16. The method for producing a haploid plant according to any one of claims 12 to 15, wherein pollen from the haploid inducer plant line contains a CRISPR-Cas9 cassette designed to target gene(s) of interest.

17. A method for generating a doubled haploid plant comprising inducing chromosome doubling in the haploid plant produced by the method defined in any one of claims 7 to 16.
